(19) Europäisches Patentamt
European Patent Office
Office européen des brevets

(11) Veröffentlichungsnummer: **0 121 112**
**B1**

(12) # EUROPÄISCHE PATENTSCHRIFT

(45) Veröffentlichungstag der Patentschrift: **13.05.87**

(51) Int. Cl.⁴: **C 07 H 19/04,** A 61 K 31/70

(21) Anmeldenummer: **84102221.3**

(22) Anmeldetag: **02.03.84**

(54) **2'-Desoxy-3'-phosphonylmethyl-nucleoside und Herstellungsverfahren.**

(30) Priorität: **04.03.83 DE 3307744**

(43) Veröffentlichungstag der Anmeldung: **10.10.84 Patentblatt 84/41**

(45) Bekanntmachung des Hinweises auf die Patenterteilung: **13.05.87 Patentblatt 87/20**

(84) Benannte Vertragsstaaten: **AT BE CH DE FR GB IT LI NL SE**

(56) Entgegenhaltungen:
**DE - A - 2 209 834**
**US - A - 3 238 191**
**US - A - 3 446 793**
**US - A - 3 558 595**

(73) Patentinhaber: **Gesellschaft für Biotechnologische Forschung mbH (GBF), Mascheroder Weg 1, D-3300 Braunschweig-Stöckheim (DE)**

(72) Erfinder: **Morr, Michael, Dipl.-Chem. Dr., Elmblick 53, D-3340 Wolfenbüttel (DE)**
Erfinder: **Kakoschke, Christel, Kuhlenkamp 5, D-3325 Lengede 4 (DE)**
Erfinder: **Fritz, Hans-Joachim, Dipl.-Chem. Dr., Classen-Kappelmannstrasse 30a, D5000 Köln 41 (DE)**

(74) Vertreter: **Boeters, Hans Dietrich, Dr. et al, Boeters, Bauer & Partner Thomas-Wimmer-Ring 14, D-8000 München 22 (DE)**

**EP 0 121 112 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Es sind bereits Phosphinylmethylderivate von Nucleosiden bekannt, die in 2'-Position des Moleküls eine Hydroxylgruppe tragen; vgl. J. Am. Chem. Soc. 92 (1970) 5510.

US-A-3238191 beschreibt Phosphonsäure-analoga von Nukleosidphosphaten, insbesondere zwei Verbindungen mit P-C-Bindungen, allerdings innerhalb einer -C3-O-P-C-Gruppierung. Unter Verwendung von derartigen Monomeren hergestellte Oligomere wären durch Nukleasen spaltbar.

Aus der DE-A-2009834.4 sind Nukleosid-6'-phosphonsäuren und Derivate bekannt, deren -C5-C-P-Gruppierung von Zellwandphosphatasen nicht angegriffen wird, die die -C5-O-P-Gruppierung freier Mononukleotide angreifen. Aus den bekannten Verbindungen hergestellte Oligomere würden allerdings an ihrer -C3-O-P-Gruppierung durch Nukleasen gespalten werden können.

Aufgabe der Erfindung ist es, biologisch aktive Verbindungen vorzusehen, die u.a. nach üblicher Modifizierung und chemischem Einbau in eine Oligonucleotidsequenz und weiterem Einbau in die DNS nicht mehr von Nucleasen, insbesondere Restriktionsenzymen gespalten werden können. Dazu werden erfindungsgemäss 2'-Desoxy-3'-phosphonylmethyl-nucleoside der folgenden allgemeinen Formel vorgesehen:

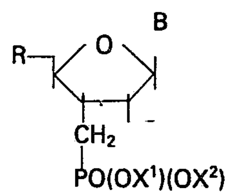

worin die folgenden Symbole die folgenden Bedeutungen besitzen:

B = Adenin, $N^6$-Benzoyladenin;

Guanin, $N^2$-Isobutyrylguanin, $N^2$-Isobutyryl-6-O-(p-nitrophenylethyl)-guanin;

Cytosin, $N^4$-Benzoylcytosin, 4-Anisoylcytosin;

Thymin, 4-O-(p-Nitrophenylethyl)-thymin; oder Uracil, und

(a)

$X^1$, $X^2$ = H, Alkalimetall, $NH_4$ oder $C_{1-4}$-Alkyl, oder

$X^1$ = Chlorphenyl, 2,2,2-Trichlorethyl oder Cyanoethyl und

$X^2$ = Triethylammonium

R = OH, $N_3$, $NH_2$, $NHR^1$, $NR^1R^2$, -O-PO($OY^1$)($OY^2$), -O-PS($OY^1$)($OY^2$),

-O-PO($OY^1$)-O-PO($OY^2$)($OY^3$),

-O-PO($OY^1$)-O-PO($OY^2$)-O-PO($OY^3$)($OY^4$), wobei

$R^1$, $R^2$ = $C_{1-4}$-Alkyl, $C_{4-7}$-Cycloalkyl, $C_{6-8}$-Alkylaryl oder $C_{6-8}$-Arylaklyl und

$Y^1$, $Y^2$, $Y^3$, $Y^4$ = H, Alkalimetall oder $NH_4$ ist, oder

R = 5'-O-Monomethoxytriphenylmethyl (5'-O-Monomethoxytrityl) oder 5'-O-(4,4'-Dimethoxytriphenylmethyl)

(5'-O-(4,4'-Dimethoxytrityl)) oder

(b)

$X^1$ = H, Alkalimetall, $NH_4$ oder $C_{1-4}$-Alkyl, wobei $X^2$ und R unter Bildung eines Ringes der Gruppierung -PO

($OX^1$)-O-$CH_2$- fehlen.

(I) Herstellung von Verbindungen der allgemeinen Formel gemäss Anspruch 1, worin die folgenden Symbole die folgenden Bedeutungen besitzen:

— B = Adenin, R = OH, $X^1$ und $X^2$ = H, Alkalimetall oder $NH_4$; oder

— B = Adenin, R = OH, $X^1$ und $X^2$ = $C_{1-4}$-Alkyl; oder

— B = Adenin, R = OH, $X^1$ = H, Alkalimetall oder $NH_4$ und

$X^2$ = $C_{1-4}$-Alkyl.

Die erfindungsgemässen Verbindungen lassen sich erfindungsgemäss dadurch herstellen, dass man

(a) von einer Verbindung der allgemeinen Formel 1

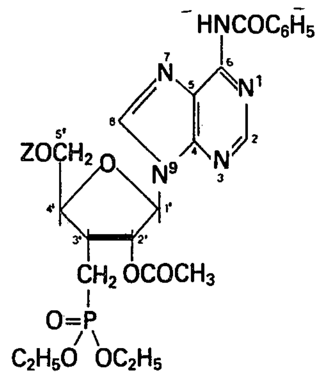

ausgeht (Z = Benzoyl oder Monomethoxytrityl), die man gemäss Moffatt in der Modifikation von Vorbrüggen [vgl. J. Am. Chem. Soc. 92 (1970) 5511 und Chem. Ber. 114 (1981) 1279] erhalten hat. Von dieser Verbindung der allgemeinen Formel 1 kann man mit einer anorganischen N-Base oder einem ihrer Salze (z.B. mit Hydroxylammoniumacetat in Pyridin) nach Ishido et al. [J.C.S. Perkin 1 (1979) 2088 oder 1 (1980) 563] selektiv die Acetylgruppe in 6-Stellung die Benzoylgruppe abspalten.

(b) Danach kann man das Reaktionsprodukt der allgemeinen Formel 2

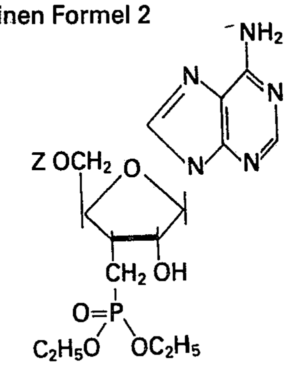

mit einer Verbindung der Formel $R^3$-CS-$R^4$ ($R^3$ = Cl, $R^4$ = Phenyl, Phenoxy; $R^3$ = $R^4$ = Imidazol-1-yl) in Gegenwart von 4-(Dialkylamino)-pyridin (z.B. 4-(Dimethylamino)-pyridin) in einem organischen Lösungsmittel (z.B. Dichlormethan) verestern [vgl. J. Org. Chem., 46 (1981) 4300 und 4843 und J.

Am. Chem. Soc. 103 (1981) 932 mit Literaturstellen].

(c) Das erhaltene Reaktionsprodukt der allgemeinen Formel 3

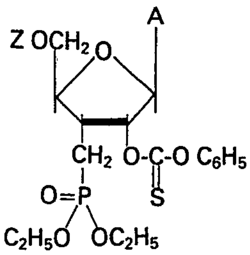

worin A einen Adeninrest bedeutet, kann man mit Tributylzinnhydrid in Toluol reduzieren, vorzugsweise bei erhöhter Temperatur, insbesondere im Bereich von 30 bis 90 °C, beispielsweise bei etwa 60 °C.

(d1) Das Reaktionsprodukt der allgemeinen Formel 4

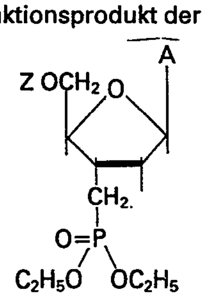

kann man mit Halogentrimethylsilan (wie Brom- oder Jodtrimethylsilan) in einem Halogenkohlenwasserstoff (wie Dichlormethan) umestern und zur freien Säure oder ihrem Ammonium- oder Alkalimetallsalz der allgemeinen Formel des Anspruchs 1 (wobei $X^1$ und $X^2$ ein Wasserstoffatom, ein Alkalimetall oder $NH_4$ und R eine OH-Gruppe bedeuten) verseifen oder

(d2) man kann von dem Reaktionsprodukt der allgemeinen Formel 4 mit Esterase (E.C. 3.1.1.1) aus Schweineleber die genannte Schutzgruppe Z (Z = Benzoyl) zum Diester der allgemeinen Formel gemäss Anspruch 1 (wobei $X^1$ und $X^2$ eine $C_{1-4}$-Alkylgruppe und R eine OH-Gruppe bedeuten) abspalten und gegebenenfalls

(d3) den Diester zum Monoester der allgemeinen Formel gemäss Anspruch 1 (wobei $X^1$ ein Wasserstoffatom, ein Alkalimetall oder $NH_4$, $X^2$ eine $C_{1-4}$-Alkylgruppe und R eine OH-Gruppe bedeuten) verseifen.

Die Stufe (a) kann man mit einer Ausbeute von 60 bis 70% und die Stufen (b), (c), (d1), (d2) und (d3) mit praktisch quantitativer Ausbeute durchführen.

(II) Herstellung von Verbindungen der allgemeinen Formel gemäss Anspruch 1, worin die folgenden Symbole die folgenden Bedeutungen besitzen:

— B = Adenin, R = $N_3$ und $X^1$ und $X^2$ = $C_{1-4}$-Alkyl; oder

— B = Adenin, R = $NH_2$, $NHR^1$ oder $NR^1R^2$ ($R^1$, $R^2$ = $C_{1-4}$-aliphatischer, $C_{4-7}$-cycloaliphatischer, $C_{6-8}$-aliphatisch/aromatischer oder $C_{6-8}$-aromatisch/aliphatischer Rest),

$X^1$ = H, Alkalimetall oder $NH_4$ und $X^2$ = $C_{1-4}$-Alkyl; oder

— B = Adenin, R = $N_3$, $NH_2$, $NHR^1$ oder $NR^1R^2$ ($R^1$, $R^2$ = $C_{1-4}$-aliphatischer, $C_{4-7}$-cycloaliphatischer, $C_{6-8}$-aliphatisch/aromatischer oder $C_{6-8}$-aromatisch/aliphatischer Rest) und

$X^1$ und $X^2$ = H, Alkalimetall oder $NH_4$.

Die erfindungsgemässen Verbindungen lassen sich erfindungsgemäss dadurch herstellen, dass man

(a) von einem Diester gemäss (I) (d2) ausgeht, den man in der 5'-Stellung aktiviert, z.B. mit Tosylchlorid (p-Toluolsulfonsäurechlorid) in Pyridin.

(b1) Danach kann man den aktivierten Diester mit einem Azid, z.B. Natriumazid in Dimethylformamid, zum Diester der allgemeinen Formel gemäss Anspruch 1 umsetzen (wobei B einen Adeninrest, R einen Azidrest und $R^1$ und $R^2$ einen $C_{1-4}$-Alkylrest bedeuten); oder

(b2) man kann den aktivierten Diester mit flüssigem Ammoniak oder einem primären oder sekundären Amin zum Halbester der allgemeinen Formel gemäss Anspruch 1 umsetzen (wobei B einen Adeninrest, R einen $NH_2$-, $NHR^1$- oder $NR^1R^2$-Rest, $R^1$ und $R^2$ einen $C_{1-4}$-aliphatischen, $C_{4-7}$-cycloaliphatischen, $C_{6-8}$-aliphatischen/aromatischen oder $C_{6-8}$-aromatisch/aliphatischen Rest, $X^1$ ein Wasserstoffatom, ein Alkalimetall oder $NH_4$ und $X^2$ einen $C_{1-4}$-Alkylrest bedeuten); und gegebenenfalls

(b3) den erhaltenen Diester oder Halbester mit Halogentrimethylsilan (z.B. Brom- oder Jodtrimethylsilan) umestern und zur freien Säure oder einem ihrer Salze der allgemeinen Formel gemäss Anspruch 1 verseifen (wobei B einen Adeninrest, R einen $N_3$-, $NH_2$-, $NHR^1$- oder $NR^1R^2$-Rest, $R^1$ und $R^2$ einen $C_{1-4}$-aliphatischen, $C_{4-7}$-cycloaliphatischen, $C_{6-8}$-aliphatisch/aromatischen oder $C_{6-8}$-aromatisch/aliphatischen Rest und $X^1$ und $X^2$ ein Wasserstoffatom, ein Alkalimetall oder $NH_4$ bedeuten).

(III) Herstellung von Verbindungen der allgemeinen Formel gemäss Anspruch 1, worin die folgenden Symbole die folgenden Bedeutungen besitzen:

— B = Adenin, R = $-O-PO(OY^1)(OY^2)$, $-O-PS(OY^1)(OY^2)$,
$-O-PO(OY^1)-O-PO(OY^2)(OY^3)$ oder
$-O-PO(OY^1)-O-PO(OY^2)-O-PO(OY^3)(OY^4)$
($Y^1$, $Y^2$, $Y^3$ und $Y^4$ = H, Alkalimetall oder $NH_4$) und $X^1$ und $X^2$ = $C_{1-4}$-Akyl; oder

— B = Adenin R = $-O-PO(OY^1)(OY^2)$, $-O-PS(OY^1)(OY^2)$,
$-O-PO(OY^1)-O-PO(OY^2)(OY^3)$ oder
$-O-PO(OY^1)-O-PO(OY^2)-O-PO(OY^3)(OY^4)$
($Y^1$, $Y^2$, $Y^3$ und $Y^4$ = H, Alkalimetall oder $NH_4$),
$X^1$ = H, Alkalimetall oder $NH_4$ und $X^2$ = $X^1$ oder $C_{1-4}$-Alkyl.

Die erfindungsgemässen Verbindungen lassen sich erfindungsgemäss dadurch herstellen, dass man

(a) von einem Diester gemäss (I) (d2) ausgeht, den man gemäss Yoshikawa et al. [Tetrahedron Lett. (1967) 5065] z.B. mit $POCl_3$ oder $PSCl_3$ in einem Phosphorsäuretrialkylester (wie Triethylphosphat) zum Diester der allgemeinen Formel gemäss Anspruch 1 phosphoryliert und zur freien 5'-Monophosphor- oder 5'-Thionophosphorsäure oder ihren Salzen verseift [wobei B einen Adeninrest, R einen -O-PO(O$Y^1$)(O$Y^2$)- oder -O-PS(O$Y^1$)(O$Y^2$)-Rest, $Y^1$ und $Y^2$ ein Wasserstoffatom, ein Alkalimetall oder $NH_4$ und $X^1$ und $X^2$ einen $C_{1-4}$-Alkylrest bedeuten]; und gegebenenfalls —

(b) das erhaltene Produkt zum Halbester oder zur freien 3'-Methylenphosphonsäure der allgemeinen Formel gemäss Anspruch 1 verseift [wobei B einen Adeninrest, R einen -O-PO(O$Y^1$)(O$Y^2$)- oder -O-PS(O$Y^1$)(O$Y^2$)-Rest, $X^1$, $Y^1$ und $Y^2$ ein Wasserstoffatom, ein Alkalimetall oder $NH_4$ und $X^2$ = $X^1$ oder einen $C_{1-4}$-Alkylrest bedeuten]; wobei man gegebenenfalls

(c) vor oder nach Stufe (b) gemäss Hoard & Ott [J. Am. Chem. Soc., 87 (1965) 1785] oder Michelson [Biochim. Biophys. Acta, 1 (1964) 91] oder enzymatisch gemäss Marutzky (Diss. TU Braunschweig 1975) die in Stufe (a) erhaltene Phosphorsäure oder ihr Salz zu Di- oder Triphosphorsäuren oder ihren Salzen der allgemeinen Formel gemäss Anspruch 1 phosphoryliert (wobei B einen Adeninrest, R einen -O-PO(O$Y^1$)-O-PO(O$Y^2$)(O$Y^3$)- oder -O-PO(O$Y^1$)-O-PO(O$Y^2$)-O-PO(O$Y^3$)(O$Y^4$)-Rest, $Y^1$, $Y^2$, $Y^3$ und $Y^4$ ein Wasserstoffatom, ein Alkalimetall oder $NH_4$ und $X^1$ ein Wasserstoffatom, ein Alkalimetall, $NH_4$ oder einen $C_{1-4}$-Alkylrest und $X^2$ einen $C_{1-4}$-Alkylrest bedeuten).

(IV) Herstellung von Verbindungen der allgemeinen Formel gemäss Anspruch 1, worin die folgenden Symbole die folgenden Bedeutungen besitzen:

— B = Adenin, R und $X^2$ fehlen unter Bildung eines Ringes der Gruppierung -PO(O$X^1$)-O-$CH_2$-, $X^1$ = H, Alkalimetall, $NH_4$ oder $C_{1-4}$-Alkyl.

Die erfindungsgemässen Verbindungen lassen sich erfindungsgemäss dadurch herstellen, dass man

(a) von einem Diester gemäss (I) (d2) ausgeht, den man mit alkalischen Katalysatoren zu einer Verbindung der allgemeinen Formel gemäss Anspruch 1 umsetzt (wobei B einen Adeninrest und $X^1$ einen $C_{1-4}$-Alkylrest bedeuten und R und $X^2$ unter Bildung eines Ringes der Gruppierung -PO(O$X^1$)-O-$CH_2$- fehlen); oder

(b) von der freien Säure oder einem ihrer Salze gemäss (I) (d1) ausgeht und diese Ausgangsverbindung (gegebenenfalls in Form seines Pyridiniumsalzes) mit Cyclisierungsmitteln (beispielsweise Dicyclohexylcarbodiimid) in einem organischen Lösungsmittel (beispielsweise Pyridin) bei erhöhter Temperatur cyclisiert; und gegebenenfalls

(c) das erhaltene Produkt der Stufe (a) mit einem Halogentrimethylsilan (beispielsweise Brom- oder Jodtrimethylsilan) in einem Halogenkohlenwasserstoff (beispielsweise Dichlormethan) umestert und zur freien Säure oder ihren Salzen der allgemeinen Formel gemäss Anspruch 1 verseift (wobei B einen Adeninrest und $X^1$ ein Wasserstoffatom, ein Alkalimetall oder $NH_4$ bedeuten und R und $X^2$ unter Bildung eines Ringes der Gruppierung -PO(O$X^1$)-O-$CH_2$- fehlen.

Verbindungen der allgemeinen Formel gemäss Anspruch 1 mit B = Guanin, Cytosin, Thymin oder Uracil lassen sich dadurch herstellen, dass man anstelle von Verbindungen der allgemeinen Formel 2 von entsprechenden Verbindungen ausgeht, die in 1'-Stellung einen Guanin-, Cytosin-, Thymin- oder Uracil-Rest tragen.

(V) Herstellung von Verbindungen der allgemeinen Formel gemäss Anspruch 1, worin folgende Symbole die folgenden Bedeutungen besitzen:

R = 5'-O-Monomethoxytrityl oder 5'-O-(4,4'-Dimethoxytrityl)

B = $N^6$-Benzoyladenin; $N^2$-Isobutyrylguanin, $N^2$-Isobutyryl-6-O-(p-nitrophenylethyl)-guanin; 4-O-(p-Nitrophenylethyl)-thymin; $N^4$-Benzoylcytosin oder 4-Anisoylcytosin

$X^1$ = 2-Chlorphenyl, 2,2,2-Trichlorethyl oder Cyanoethyl

$X^2$ = Triethylammonium.

Diese erfindungsgemässen Verbindungen, z.B. solche der allgemeinen Formel 7

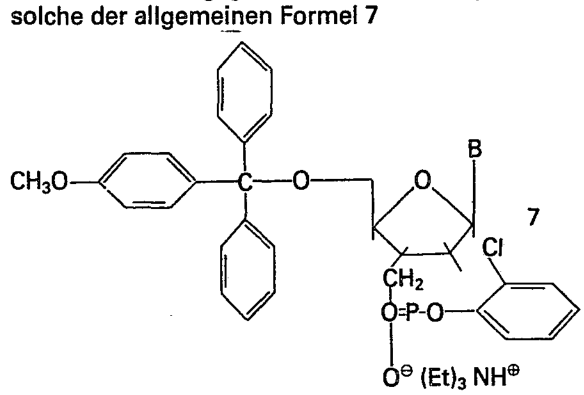

7

können wie folgt hergestellt werden:

(a) Eine Verbindung der folgenden allgemeinen Formel 8, hergestellt wie oben unter (I) beschrieben, wobei die Schutzgruppe Z in Stufe (d2) abgespalten worden ist,

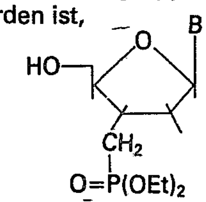

8

kann mit 4-Monomethoxytriphenylmethylchlorid (Methoxytritylchlorid) in Gegenwart von 4-Dimethylaminopyridin umgesetzt werden; vgl. Y. Lapidot und H.G. Khorana, J. Am. Chem. Soc. 85, 3862 (1963).

(b) Die erhaltene Verbindung der allgemeinen Formel 9

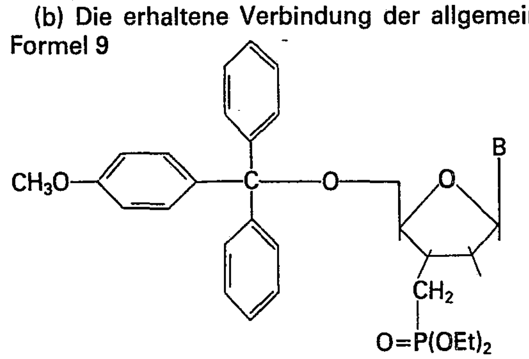

9

kann man mit Halogentrimethylsilan umsetzen, und zwar entweder mit

(b1) Chlortrimethylsilan in Gegenwart von Natriumjodid und Acetonitril gemäss T. Morita, Y. Okamoto und H. Sakurai, Tetrahedron Letters, No. 28, Seiten 2523–2526 (1978), oder

(b2) Bromtrimethylsilan gemäss C.E. McKenna, H.T. Higa, N.H. Higa und M. McKenna, Tetrahedron Letters, 977 (1977).

(c) Das Reaktionsprodukt der allgemeinen Formel 10

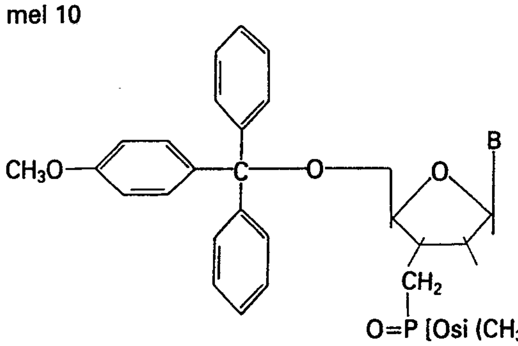

10

wird mit Methanol verseift und an DEAE-Sephadex chromatographiert.

(d) Das Reaktionsprodukt der allgemeinen Formel 11

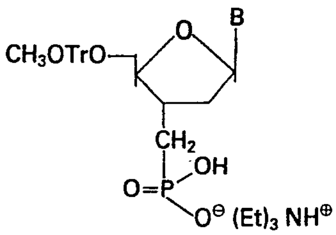

11

wird dann unter Verwendung von 2,4,6-Triisopropylbenzolsulfochlorid in Pyridin aktiviert und mit 2-Chlorphenol in an sich bekannter Weise zur Verbindung der allgemeinen Formel 7 verestert.

Die erfindungsgemässen Verbindungen können beim rein chemischen Aufbau von Oligonucleotiden im Phospho(r)triester-Verfahren verwendet werden:

An der folgenden Modellsubstanz, Isobutylphosphonsäure-monoester von Desoxyadenosin

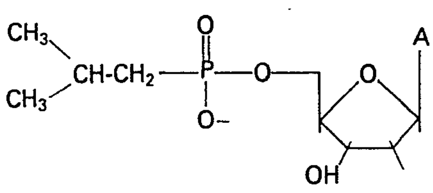

12

wurde der Syntheseweg erprobt:

(alpha) Isobutylphosphonsäure der Formel 13

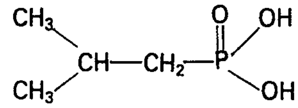

13

wird mit Triisopropylbenzolsulfochlorid in Pyridin mit 2-Chlorphenol in an sich bekannter Weise umgesetzt und dann an DEAE-Sephadex chromatographiert.

(beta) Das Reaktionsprodukt der Formel 14

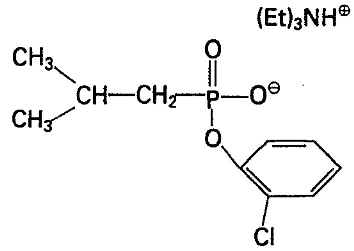

14

wird mit MSNT (1-(Mesitylensulfonyl)-3-nitro-1,2,4-triazol) als Kondensationsmittel in Pyridin und Desoxy-3'-benzoyloxy-$N^6$-benzoyl-adenosin umgesetzt.

(gamma) Der erhaltene gemischte Isobutylphosphonsäurediester der Formel 15

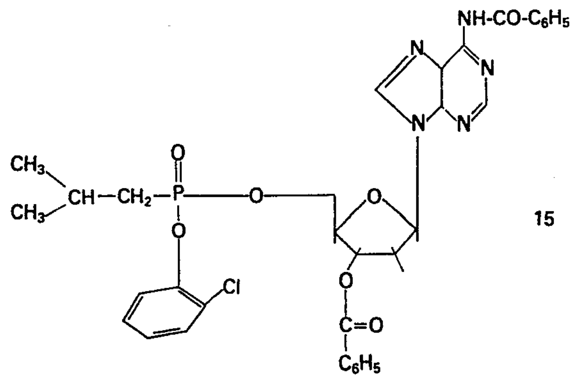

15

wird mit konzentriertem Ammoniak zum Desoxyadenosin-isobutylphosphonsäuremonoester der Formel 12 verseift.

Nachstehend wird die Erfindung durch Beispiele näher erläutert.

9-[5-O-Benzoyl-3-desoxy-3-(diethoxyphosphonylmethyl)-β-D-ribofuranosyl]-adenin oder 5'-O-Benzoyl-3'-diethoxyphosphonylmethyl-3'-desoxyadenosin) (2)

1,134 g (1,74 mmol) 1 werden in 35 ml Pyridin mit ca. 9 mmol Hydroxylammoniumacetat ($NH_2OH.HAc$) gelöst und 45 h bei Raumtemperatur stehengelassen.

Dünnschichtchromatografische Untersuchung einer Probe und anschliessende Auswertung mit

einem DC-Scanner zeigt folgendes Ergebnis:

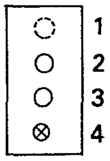

1 = 1 0%

2 = 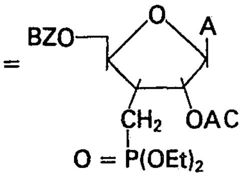

14%

3 = 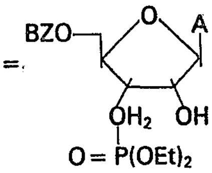 2

70%

4 = 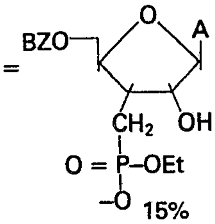

15%

Nach Abziehen der flüchtigen Bestandteile im Ölpumpenvakuum (Pyridin und überschüssiges Hydroxylammoniumacetat) wird der Rückstand in Chloroform/Methanol 9:1 gelöst und auf eine Kieselgelsäule (25×5 cm, Kieselgel 60, Korngrösse 0,063–0,200 mm, Merck, Darmstadt) gegeben. Nach der Elution mit dem gleichen Laufmittel und Eindampfen des 2-haltigen Peaks erhält man 580 mg (ca. 65%) Elution mit Methanol liefert den Halbester von 2.

9-[5-O-Benzoyl-3-desoxy-3-(diethoxyphosphonylmethyl)-2-O-phenoxythiocarbonyl-β-D-ribofuranosyl]-adenin (3) oder 5′-O-Benzoyl-3′-diethoxyphosphonylmethyl-2′-O-phenoxythiocarbonyl-3′-desoxyadenosin

420 mg (0,83 mmol) 2 werden in 20 ml wasserfreiem Dichlormethan (Robins et al., Arbeiten in Acetonitril – ist im obigen Fall nicht anwendbar, da das Ausgangsprodukt nach geraumer Zeit auskristallisiert) gelöst und unter Feuchtigkeitsausschluss mit 0,21 ml (1,5 mmol) Thiokohlensäure-O-phenylester-chlorid und 270 mg (2,2 mmol) 4-Dimethylaminopyridin versetzt. Nach 12stündigem Rühren bei Raumtemperatur ist die Umsetzung vollständig (DC, Kieselgel, Laufmittel: Dichlormethan/Methanol 9:1). Es wird zur Trockne eingeengt und der Rückstand zwischen Essigsäurerethylester (EE) und 1 m $KH_2PO_4$-Lösung (pH 4) verteilt. Nach dem Trocknen der EE-Phase mit wasserfreiem Natriumsulfat wird zur Trockne eingeengt, der Rückstand in wenig Dichlormethan/Methanol 9:1 gelöst und über eine Kieselgelsäule (10×5 cm) chromatografiert. Nach dem Einengen der 3-haltigen Fraktion erhält man 445 mg (67%).

9-[2-Desoxy-5-O-benzoyl-3-desoxy-3-(diethoxyphosphonylmethyl)-β-D-ribofuranosyl]-adenin (4) oder 5′-O-Benzoyl-3′-desoxy-3′-diethoxyphosphonylmethyl-2′-desoxyadenosin

190 mg (0,29 mmol) 3 werden in 6 ml wasserfreiem Toluol gelöst und mit 0,155 ml Tributylzinnhydrid und 32 mg 2,2′-Azobis-(2-methylpropionitril) bzw. (α,α-Azo-iso-butyronitril) versetzt. Nach dem Erwärmen auf 70 °C unter Feuchtigkeitsausschluss wird die Reaktion nach 7 h beendet. Nach dem Einengen zur Trockne wird in wenig Chloroform/Methanol 9:1 gelöst und die Lösung auf eine Kieselgelsäule (24×2 cm) gegeben. Nach dem Eluieren mit dem obigen Laufmittel wird der 4-haltige Peak eingeengt. Ausbeute: 123 mg (86%).

9-[2-Desoxy-3-desoxy-3-(dihydroxyphosphonylmethyl)-β-D-ribofuranosyl]-adenin oder 2′-Desoxy-3′-dihydroxyphosphonylmethyl-3′-desoxyadenosin (5)

166 mg (0,34 mmol) 4 werden in 4 ml wasserfreiem Dichlormethan gelöst und mit 0,27 ml (1,7 mmol) Trimethylbromsilan versetzt. Man lässt über Nacht rühren (im DC, LM: Chloroform/Methanol 9:1 war kein Ausgangsprodukt mehr vorhanden) und engt im Vakuum ein. Der Rückstand wird mit Wasser hydrolysiert und die wässrige schwachsaure Lösung zur Trockne eingeengt. Zur Abspaltung der 5′-O-Benzoyl-Schutzgruppe lässt man 5 Tage in gesättigter methanolischer Ammoniaklösung (25 ml) stehen. Nach dem Abziehen zur Trockne wird der Rückstand in 10 ml Wasser gelöst, auf pH 7,5 eingestellt, auf eine DEAE-Sephadex-Säule ($HCO_3$-Form, 30×25 cm) gegeben und mit einem Gradienten aus 1 l Wasser/1 l 0,4 m TEAB-Puffer chromatografiert. Nach dem Einengen des 5-haltigen Peaks und wiederholtem Eindampfen mit Methanol wird die wässrige Lösung über eine Dowex-Natrium-Säule gegeben und anschliessend lyophilisiert.

9-[2-Desoxy-3-desoxy-3-(diethoxyphosphonylmethyl)-β-D-ribofuranosyl]-adenin oder 2′-Desoxy-3′-diethoxyphosphonylmethyl-3′-desoxyadenosin (6)

8 mg (16 μmol) 4 werden in 4 ml Wasser gelöst und mit gesättigter Natriumhydrogencarbonatlösung auf pH 8,2 eingestellt. Nach der Zugabe von 10 μl Esterase (Boehringer) lässt man unter pH-Wert-Kontrolle 24 h bei 37 °C rühren. Die Umsetzung wird dünnschichtchromatografisch verfolgt (DC, Kieselgel, LM: Chloroform/Methanol 8:2). Nach dem Einengen zur Trockne und Extraktion mit dem Laufmittel wird auf einer kleinen Kieselgelsäule chromatografiert. Ausbeute: 5,2 mg (83%).

Die Strukturen aller Verbindungen sind durch $^1H$-, $^{13}C$- und $^{31}P$-Spektren sowie chromatografische Untersuchungen einwandfrei abgesichert.

Abkürzungen

DC Dünnschichtchromatographie

LM Laufmittel

TEAB-Puffer Triethylammoniumhydrogencarbonat-Puffer

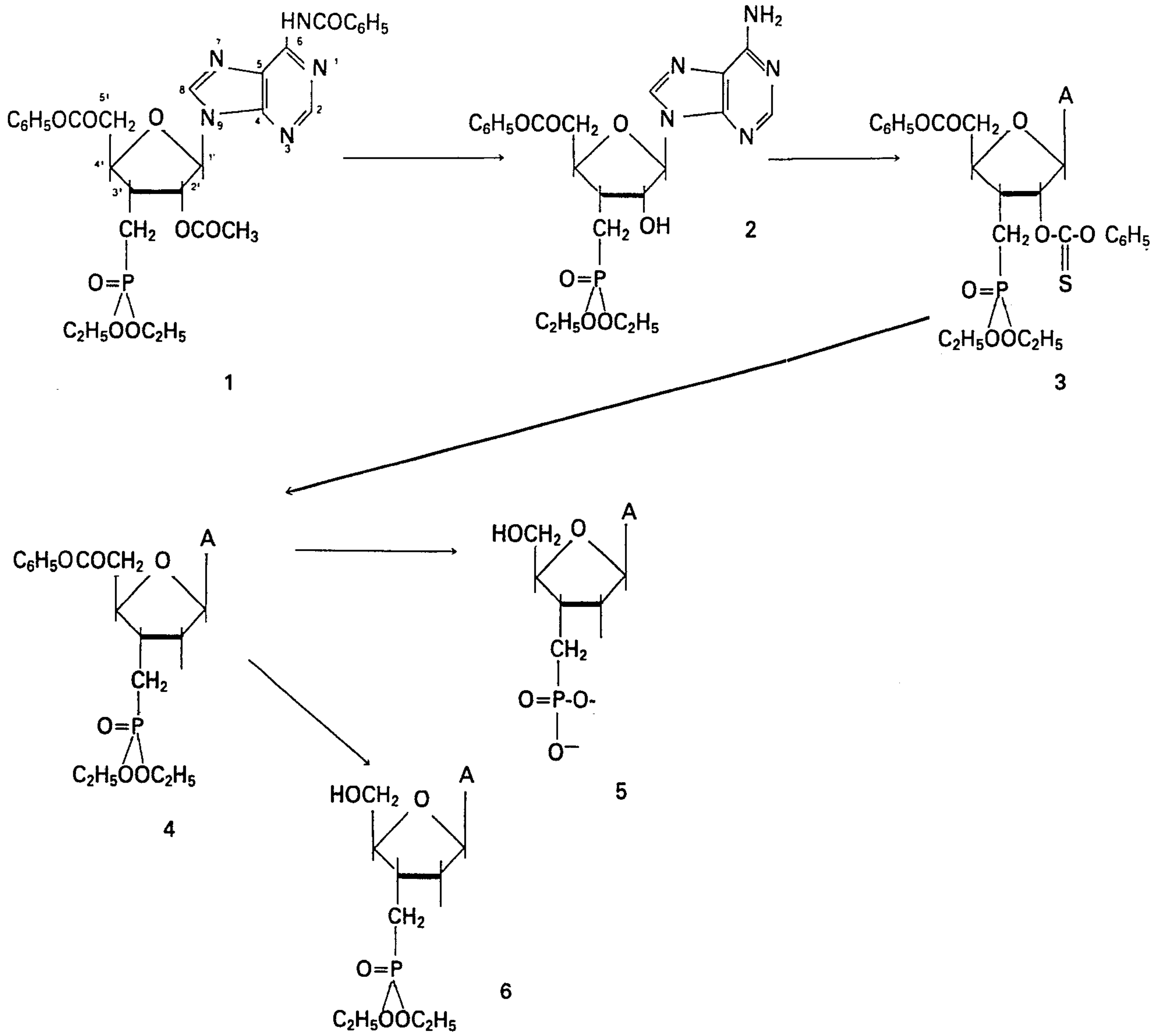

1 2 3 4 5 6

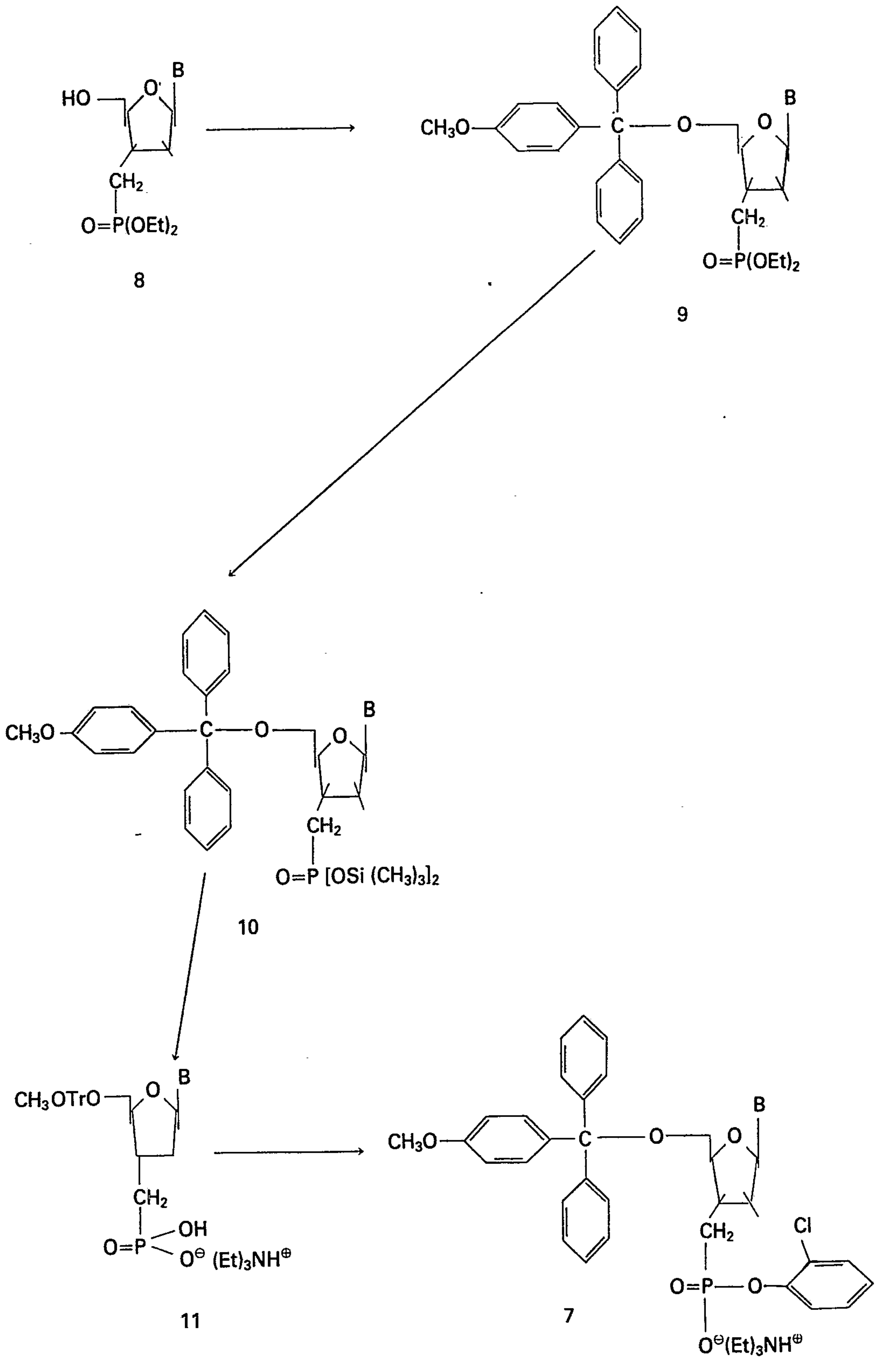

8

9

10

11

7

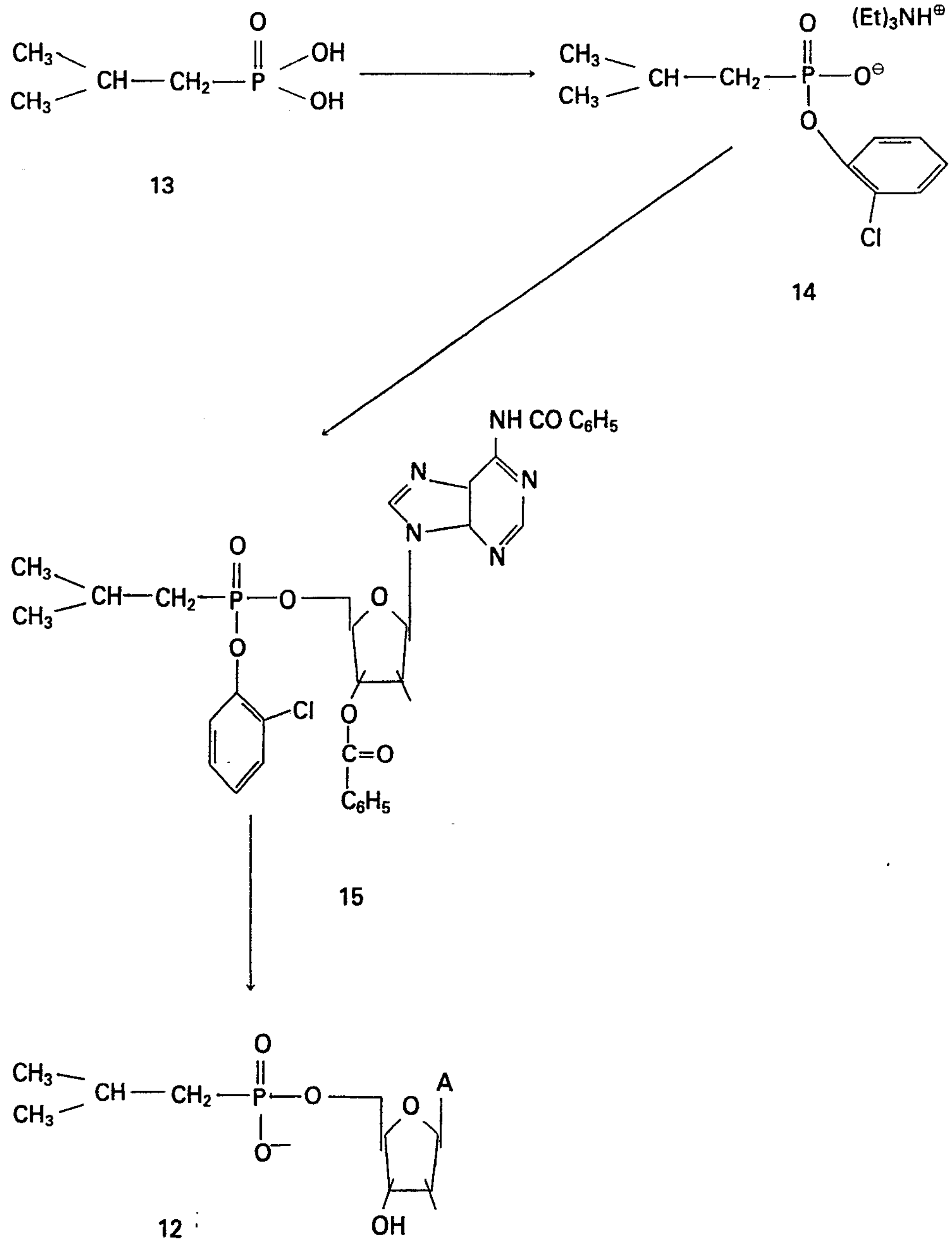

13

14

15

12

## Patentansprüche

1. 1. 2'-Desoxy-3'-phosphonylmethyl-nucleoside der folgenden allgemeinen Formel

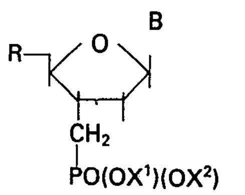

(I)

worin die folgenden Symbole die folgenden Bedeutungen besitzen:

B = Adenin, $N^6$-Benzoyladenin;
Guanin, $N^2$-Isobutyrylguanin, $N^2$-Isobutyrtyl-6-O-(p-nitrophenylethyl)-guanin;
Cytosin, $N^4$-Benzoylcytosin, 4-Anisoylcytosin;
Thymin, 4-O-(p-Nitrophenylethyl)-thymin; oder
Uracil und

(a) $X^1$, $X^2$ = H, Alkalimetall, $NH_4$ oder $C_{1-4}$-Alkyl, oder
$X^1$ = Chlorphenyl, 2,2,2-Trichlorethyl oder Cyanoethyl und
$X^2$ = Triethylammonium,
R = OH, $N_3$, $NH_2$, $NHR^1$, $NR^1R^2$, -O-PO($OY^1$)($OY^2$), -O-PS($OY^1$)($OY^2$),
-O-PO($OY^1$)-O-PO($OY^2$)($OY^3$),
-O-PO($OY^1$)-O-PO($OY^2$)-O-PO($OY^3$)($OY^4$), wobei
$R^1$, $R^2$ = $C_{11-4}$-Alkyl, $C_{4-7}$-Cycloalkyl, $C_{6-8}$-Alkylaryl oder $C_{6-8}$-Arylalkyl und

$Y^1$, $Y^2$, $Y^3$, $Y^4$ = H, Alkalimetall oder $NH_4$ ist, oder
R = 5'-O-Monomethoxytriphenylmethyl (5'-O-Monomethoxytrityl) oder 5'-O-(4,4'-Dimethoxytriphenylmethyl) (5'-O-(4,4'-Dimethoxytrityl)) oder
(b) $X^1$ = H, Alkalimetall, $NH_4$ oder $C_{1-4}$-Alkyl, wobei
$X^2$ und R unter Bildung eines Ringes der Gruppierung -PO ($OX^1$)-O-$CH_2$ fehlen.

2. Verfahren zur Herstellung der 2'-Desoxy-3'-phosphonylmethyl-nucleoside der folgenden allgemeinen Formel (I) gemäss Anspruch 1,

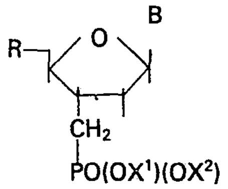

(I)

wobei die folgenden Symbole die folgenden Bedeutungen besitzen

— B = Adenin, Guanin, Cytosin, Thymin oder Uracil
R = OH, $X^1$ und $X^2$ = H, Alkalimetall oder $NH_4$;
oder

— B = Adenin, Guanin, Cytosin, Thymin oder Uracil
R = OH, $X^1$ und $X^2$ = $C_{1-4}$-Alkyl; oder

— B = Adenin, Guanin, Cytosin, Thymin oder Uracil
R = OH, $X^1$ = H, Alkalimetall oder $NH_4$ und $X^2$ = $C_{1-4}$-Alkyl,

dadurch gekennzeichnet, dass man

(a) von einer Verbindung der allgemeinen Formel 1

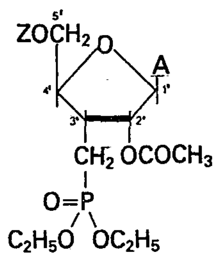

in der A ein N-geschützter Adenin-, Guanin-, Cytosin-, Thymin- oder Uracilrest und Z Benzoyl oder Monomethoxytrityl ist, in an sich bekannter Weise mit einer anorganischen N-Base oder einem ihrer Salze selektiv die Acetylgruppe abspaltet,

(b) das Reaktionsprodukt der allgemeinen Formel 2

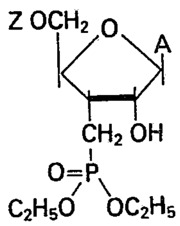

in der A ein gegebenenfalls N-geschützter Adenin-, Guanin-, Cytosin-, Thymin- oder Uracilrest ist, mit einer Verbindung der Formel $R^3$-CS-$R^4$ ($R^3$ = Cl, $R^4$ = Phenyl, Phenoxy; $R^3$ = $R^4$ = Imidazol-1-yl) in Gegenwart von 4-Dialkylamino-pyridin in einem organischen Lösungsmittel in an sich bekannter Weise verestert,

(c) das erhaltene Reaktionsprodukt der allgemeinen Formel 3

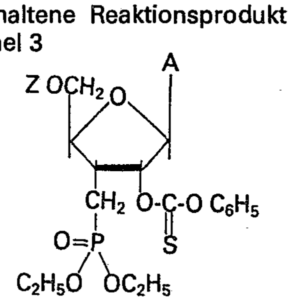

mit Tributylzinnhydrid in einem inerten aromatischen Lösungsmittel reduziert,

(d1) das Reaktionsprodukt der allgemeinen Formel 4

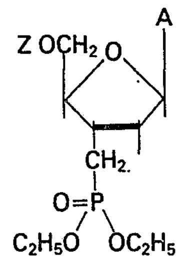

mit Halogentrimethylsilan in einem Halogenkohlenwasserstoff umestert und zur freien Säure oder ihrem Ammonium- oder Alkalimetallsalz der allgemeinen Formel (I) (wobei $X^1$ und $X^2$ ein Wasserstoffatom, ein Alkalimetall oder $NH_4$ und R eine OH-Gruppe bedeuten) verseift oder

(d2) von dem Reaktionsprodukt der allgemeinen Formel 4 mit Esterase (E.C. 3.1.1.1.) aus Schweineleber die genannte Schutzgruppe Z zum Diester der allgemeinen Formel (I) (wobei $X^1$ und $X^2$ eine $C_{1-4}$-Alkylgruppe und R eine OH-Gruppe bedeuten) abspaltet und gegebenenfalls

(d3) den Diester zum Monoester der allgemeinen Formel (I) (wobei $X^1$ ein Wasserstoffatom, ein Alkalimetall oder $NH_4$, $X^2$ eine $C_{1-4}$-Alkylgruppe und R eine OH-Gruppe bedeuten) verseift.

3. Verfahren zur Herstellung von Verbindungen der allgemeinen Formel (I) gemäss Anspruch 1, worin die folgenden Symbole die folgenden Bedeutungen besitzen:

— B = Adenin, Guanin, Cytosin, Thymin oder Uracil,
R = $N_3$ und $Y^1$ und $X^2$ = $C_{1-4}$-Alkyl; oder

— B = Adenin, Guanin, Cytosin, Thymin oder Uracil,
R = $NH_2$, $NHR^1$ oder $NR^1R^2$ ($R^1$, $R^2$ = $C_{1-4}$-aliphatischer, $C_{4-7}$-cycloaliphatischer, $C_{6-8}$-aliphatisch/aromatischer oder $C_{6-8}$-aromatisch/aliphatischer Rest),
$X^1$ = H, Alkalimetall oder $NH_4$ und $X^2$ = $C_{1-4}$-Alkyl; oder

— B = Adenin, Guanin, Cytosin, Thymin oder Uracil,
R = $N_3$, $NH_2$, $NHR^1$ oder $NR^1R^2$ ($R^1$, $R^2$ = $C_{1-4}$-aliphatischer, $C_{4-7}$-cycloaliphatischer, $C_{6-8}$-aliphatisch/aromatischer, oder $C_{6-8}$-aromatisch/aliphatischer Rest) und $X^1$ und $X^2$ = H, Alkalimetall oder $NH_4$,

dadurch gekennzeichnet, dass man

(a) einen gemäss Anspruch 2, Stufe (d2) hergestellten Diester in der 5'-Stellung aktiviert,

(b1) den aktivierten Diester mit einem Azid zum Diester der allgemeinen Formel (I) umsetzt (wobei B einen Adenin-, Guanin-, Cytosin-, Thymin- oder Uracilrest, R einen Azidrest und $R^1$ und $R^2$ einen $C_{1-4}$-Alkylrest bedeuten); oder

(b2) den aktivierten Diester mit flüssigem Ammoniak oder einem primären oder sekundären Amin zum Halbester der allgemeinen Formel (I) umsetzt (wobei B einen Adenin-, Guanin-, Cytosin-, Thymin- oder Uracilrest, R einen $NH_2$-, $NHR^1$- oder $NR^1R^2$-Rest, $R^1$ und $R^2$ einen $C_{1-4}$-aliphatischen, $C_{4-7}$-cycloaliphatischen, $C_{6-8}$-aliphatisch/aromatischen oder $C_{6-8}$-aromatisch/aliphatischen Rest, $X^1$ ein Wasserstoffatom, ein Alkalimetall oder $NH_4$ und $X^2$ einen $C_{1-4}$-Alkylrest bedeuten); und gegebenenfalls

(b3) den erhaltenen Diester oder Halbester mit Halogentrimethylsilan umestert und zur freien Säure oder einem ihrer Salze der allgemeinen Formel (I) verseift (wobei B einen Adenin-, Guanin-, Cytosin-, Thymin- oder Uracilrest, R einen $N_3$-, $NH_2$-, $NHR^1$- oder $NR^1R^2$-Rest, $R^1$ und $R^2$ einen $C_{1-4}$-aliphatischen, $C_{4-7}$-cycloaliphatischen, $C_{6-8}$-aliphatisch/aromatischen oder $C_{6-8}$-aromatisch/aliphatischen Rest und $X^1$ und $X^2$ ein Wasserstoffatom, ein Alkalimetall oder $NH_4$ bedeuten).

4. Verfahren zur Herstellung von Verbindungen der allgemeinen Formel (I) gemäss Anspruch 1, worin die folgenden Symbole die folgenden Bedeutungen besitzen:

— B = Adenin, Guanin, Cytosin, Thymin oder Uracil,

R = -O-PO(OY$^1$)(OY$^2$), -O-PS(OY$^1$)(OY$^2$), -O-PO(OY$^1$)-O-PO(OY$^2$)(OY$^3$) oder -O-PO(OY$^1$)-O-PO(OY$^2$)-O-PO(OY$^3$)(OY$^4$)

($Y^1$, $Y^2$, $Y^3$ und $Y^4$ = H, Alkalimetall oder $NH_4$) und

$X^1$ und $X^2$ = $C_{1-4}$Alkyl; oder

$X^1$ = H, Alkalimetall oder $NH_4$ und $X^2$ = $X^1$ oder $C_{1-4}$-Alkyl,

dadurch gekennzeichnet, dass man

(a) einen gemäss Anspruch 2, Stufe (d2) hergestellten Diester in an sich bekannter Weise mit $POCl_3$ oder $PSCl_3$ in einem Phosphorsäuretrialkylester zum Diester der allgemeinen Formel (I) phosphoryliert und zur freien 5'-Monophosphor- oder 5'-Thionophosphorsäure oder ihren Salzen verseift (wobei B einen Adenin-, Guanin-, Cytosin-, Thymin- oder Uracilrest, R einen -O-PO(OY$^1$)(OY$^2$)- oder -O-PS(OY$^1$)(OY$^2$)-Rest, $Y^1$ und $Y^2$ ein Wasserstoffatom, ein Alkalimetall oder $NH_4$ und $X^1$ und $X^2$ einen $C_{1-4}$-Alkylrest bedeuten); und gegebenenfalls

(b) das erhaltene Produkt zum Halbester oder zur freien 3'-Methylenphosphonsäure der allgemeinen Formel (I) verseift (wobei B einen Adenin-, Guanin-, Cytosin-, Thymin- oder Uracil-Rest, R einen -O-PO(OY$^1$)(OY$^2$)- oder -O-PS(OY$^1$)(OY$^2$)-Rest, $X^1$, $Y^1$ und $Y^2$ ein Wasserstoffatom, ein Alkalimetall oder $NH_4$ und $X^2$ = $X^1$ oder einen $C_{1-4}$-Alkylrest bedeuten); wobei man gegebenenfalls

(c) vor oder nach Stufe (b) in an sich bekannter Weise chemisch oder enzymatisch die in Stufe (a) erhaltene Phosphorsäure oder ihr Salz zu Di- oder Triphosphorsäuren oder ihren Salzen der allgemeinen Formel (I) phosphoryliert (wobei B einen Adenin-, Guanin-, Cytosin-, Thymin- oder Uracilrest, R einen -O-PO(OY$^1$)-O-PO(OY$^2$)(OY$^3$)- oder -O-PO(OY$^1$)-O-PO(OY$^2$)-O-PO(OY$^3$)(OY$^4$)-Rest, $Y^1$, $Y^2$, $Y^3$ und $Y^4$ ein Wasserstoffatom, ein Alkalimetall oder $NH_4$ und $X^1$ ein Wasserstoffatom, ein Alkalimetall, $NH_4$ oder einen $C_{1-4}$-Alkylrest und $X^2$ einen $C_{1-4}$-Alkylrest bedeuten).

5. Verfahren zur Herstellung von Verbindungen der allgemeinen Formel (I) gemäss Anspruch 1, worin die folgenden Symbole die folgenden Bedeutungen besitzen:

— B = Adenin, Guanin, Cytosin, Thymin oder Uracil,

R und $X^2$ fehlen unter Bildung eines Ringes der Gruppierung

-PO(OX$^1$)-O-$CH_2$-, $X^1$ = H, Alkalimetall, $NH_4$ oder $C_{1-4}$-Alkyl,

dadurch gekennzeichnet, dass man

(a) einen gemäss Anspruch 2, Stufe (d2) hergestellten Diester mit alkalischen Katalysatoren zu einer Verbindung der allgemeinen Formel (I) umsetzt (wobei B einen Adenin-, Guanin-, Cytosin-, Thymin- oder Uracilrest und $X^1$ einen $C_{1-4}$-Alkylrest bedeuten und R und $X^2$ unter Bildung eines Ringes der Gruppierung -PO(OX$^1$)-O-$CH_2$- fehlen); oder

(b) die gemäss Anspruch 2, Stufe (d1) hergestellte freie Säure oder eines ihrer Salze mit Cyclisierungsmitteln in einem organischen Lösungsmittel bei erhöhter Temperatur cyclisiert; und gegebenenfalls

(c) das erhaltene Produkt der Stufe (a) mit einem Halogentrimethylsilan in einem Halogenkohlenwasserstoff umestert und zur freien Säure oder ihren Salzen der allgemeinen Formel (I) verseift (wobei B einen Adenin-, Guanin-, Cytosin-, Thymin- oder Uracilrest unc $X^1$ ein Wasserstoffatom, ein Alkalimetall oder $NH_4$ bedeuten und R und $X^2$ unter Bildung eines Ringes der Gruppierung -PO(OX$^1$)-O-$CH_2$- fehlen.

6. Verfahren zur Herstellung von Verbindungen der allgemeinen Formel (I) gemäss Anspruch 1, worin folgende Symbole die folgenden Bedeutungen besitzen:

R = 5'-O-Monomethoxytrityl oder 5'-O-(4,4'-Dimethoxytrityl)

B = $N^6$-Benzoyladenin; $N^2$-Isobutyrylguanin, $N^2$-Isobutyryl-6-O-(p-nitrophenylethyl)-guanin; 4-O-(p-Nitrophenylethyl)-thymin; $N^4$-Benzoylcytosin oder 4-Anisoylcytosin

$X^1$ = 2-Chlorphenyl, 2,2,2-Trichlorethyl oder Cyanoethyl

$X^2$ = Triethylammonium

dadurch gekennzeichnet, dass man

(a) eine gemäss Anspruch 2, Stufe (d2) hergestellte Verbindung der folgenden allgemeinen Formel 8

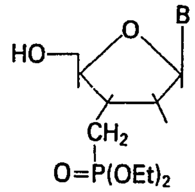

8

in der B die angegebene Bedeutung hat, in an sich bekannter Weise mit 4-Monomethoxytriphenylmethylchlorid (Methoxytritylchlorid) in Gegenwart von 4-Dimethylaminopyridin umgesetzt,

(b) die erhaltene Verbindung der allgemeinen Formel 9

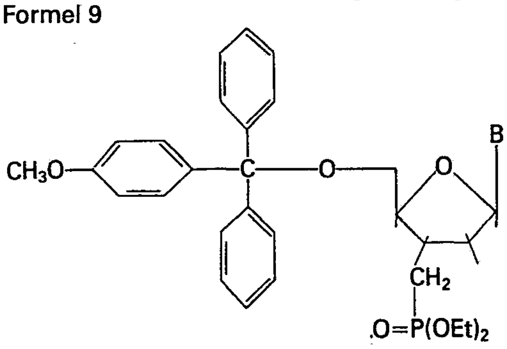

9

in an sich bekannter Weise mit Halogentrimethylsilan umsetzt,

(c) das Reaktionsprodukt der allgemeinen Formel 10

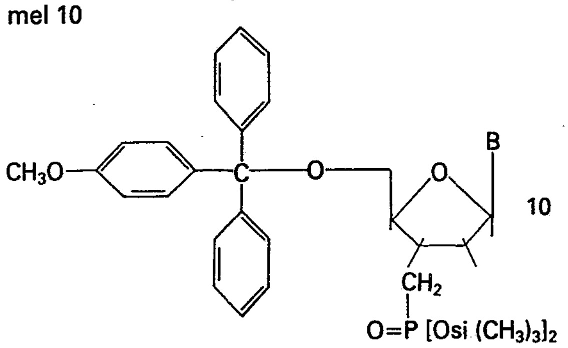

10

mit Methanol verseift-und an DEAE-Sephadex® chromatographiert,

(d) das Reaktionsprodukt der allgemeinen Formel 11

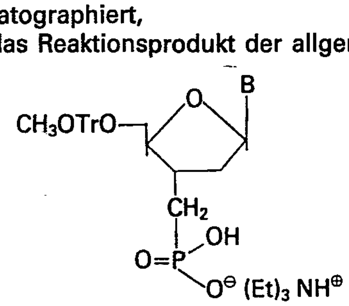

11

unter Verwendung von 2,4,6-Triisopropylbenzolsulfochlorid in Pyridin (Aktivierung) mit 2-Chlorphenol in an sich bekannter Weise zur Verbindung der allgemeinen Formel 7 verestert.

**Revendications**

1. Désoxy-2'-phosphonylméthyl-3'-nucléosides de formule générale

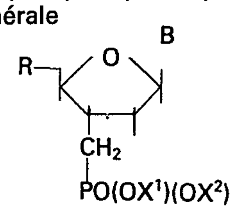

(I)

dans laquelle les symboles suivants ont les significations suivantes:

B = adénine, $N^6$-benzoyladénine;

Guanine, $N^2$-isobutyrylguanine, $N^2$-isobutyryl-O-(p-nitrophényléthyl)-6-guanine;

Cytosine, $N^4$-benzoylcytosine, anisoyl-4-cytosine; Thymine, O-(p-nitrophényléthyl)-4-thymine; ou Uracile, et

(a) $X^1$, $X^2$ = H, un métal alcalin, $NH_4$ ou un radical alkyle en $C_{1-4}$, ou

$X^1$ = le radical chlorophényle, trichloro-2,2,2-éthyle ou cyanoéthyle, et

$X^2$ = triéthylammonium;

R = OH, $N_3$, $NH_2$, $NHR^1$, $NR^1R^2$, $-O-PO(OY^1)(OY^2)$, $-O-PS(OY^1)(OY^2)$, $-O-PO(OY^1)-O-PO(OY^2)(OY^3)$, $-O-PO(OY^1)-O-PO(OY^2)-O-PO(OY^3)(OY^4)$, avec

$R^1$, $R^2$ = un groupe alkyle en $C_{1-4}$, cycloalkyle en $C_{4-7}$, alkylaryle en $C_{6-8}$ ou arylalkyle en $C_{6-8}$ et

$Y^1$, $Y^2$, $Y^3$, $Y^4$ = H, un métal alcalin ou $NH_4$, ou bien

R = O-monométhoxytriphénylméthyle-5' (O-monométhoxytrityle-5') ou O-(diméthoxy-4,4'-triphénylméthyl)-5' (O-(diméthoxy-4,4'-trityle)-5') ou bien

(b) $X^1$ = H, un métal alcalin, $NH_4$ ou un groupe alkyle en $C_{1-4}$, et $X^2$ et R manquent avec formation d'un cycle par le groupement $-PO(OX^1)-O-CH_2-$.

2. Procédé pour la préparation de désoxy-2'-phosphonylméthyl-3'-nucléosides ayant la formule générale suivante I selon la revendication 1,

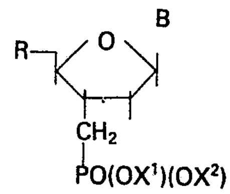

(I)

dans laquelle les symboles suivants ont les significations suivantes:

B = adénine, guanine, cytosine, thymine ou uracile,

R = OH, $X^1$ et $X^2$ = H, un métal alcalin ou $NH_4$; ou bien

B = adénine, guanine, cytosine, thymine ou uracile,

R = OH, $X^1$ et $X^2$ = un groupe alkyle en $C_{1-4}$; ou bien

B = adénine, quanine, cytosine, thymine ou uracile,

R = OH, $X^1$ = H, un métal alcalin ou $NH_4$, et

$X^2$ = un groupe alkyle en $C_{1-4}$,

caractérisé en ce que

(a) on élimine sélectivement, d'une manière connue en soi, avec une base azotée inorganique ou l'un de ses sels, le groupe acétyle d'un composé de formule générale 1

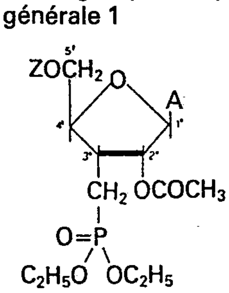

dans laquelle A est un résidu adénine, guanine, cytosine, thymine ou uracile N-protégé et Z est le radical benzoyle ou monométhoxytrityle,

(b) on estérifie d'une manière connue en soi, en présence de dialkylamino-4-pyridine dans un solvant organique, le produit de réaction de formule générale 2

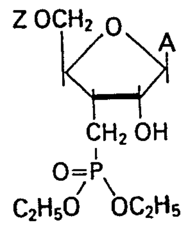

dans laquelle A est un résidu adénine, guanine, cytosine, thymine ou uracile éventuellement N-protégé, avec un composé de formule $R^3$-CS-$R^4$ ($R^3$ = Cl, $R^4$ = phényle, phénoxy; $R^3$ = $R^4$ = imidazolyle-1),

(c) on réduit le produit de réaction obtenu, de formule générale 3

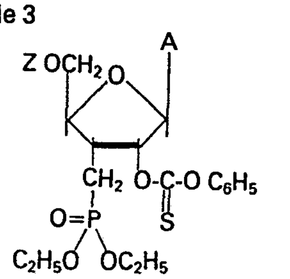

avec de l'hydrure de tributylétain dans un solvant aromatique inerte,

(d1) on transestérifie le produit de réaction de formule générale 4

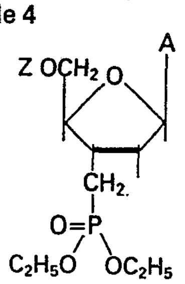

avec un halogénotriméthylsilane dans un hydrocarbure halogéné, et on le saponifie en acide libre ou en son sel d'ammonium ou de métal alcalin de formule générale (I) (où $X^1$ et $X^2$ sont chacun un atome d'hydrogène, un métal alcalin ou $NH_4$, et R est un groupe OH), ou bien

(d2) on élimine du produit de réaction de formule générale 4, avec de l'Estérase (E.C. 3.1.1.1.) tirée de foie de porc, le groupe protecteur mentionné Z, pour obtenir le diester de formule générale (I) (où $X^1$ et $X^2$ sont chacun un groupe alkyle $C_{1-4}$ et R est un groupe (OH), et éventuellement

(d3) on saponifie le diester pour obtenir le monoester de formule générale (I) (dans laquelle $X^1$ est un atome d'hydrogène, un métal alcalin ou $NH_4$, $X^2$ est un groupe alkyle en $C_{1-4}$ et R est un groupe OH).

3. Procédé pour la préparation de composés de formule générale (I) selon la revendication 1, dans laquelle les symboles suivants ou les significations suivantes:

B = adénine, guanine, cytosine, thymine ou uracile,
R = $N_3$, et $X^1$ et $X^2$ = un groupe alkyle en $C_{1-4}$; ou bien

B = adénine, guanine, cytosine, thymine ou uracile,
R = $NH_2$ $NHR^1$ ou $NR^1R^2$ ($R^1$, $R^2$ = un radical aliphatique en $C_{1-4}$, cycloaliphatique en $C_{4-7}$, aliphatique/aromatique en $C_{6-8}$ ou aromatique/aliphatique en $C_{6-8}$),
$X^1$ = H, un métal alcalin ou $NH_4$, et $X^2$ = un groupe alkyle en $C_{1-4}$; ou bien

B = adénine, guanine, cytosine, thymine ou uracile,
R = $N_3$, $NH_2$, $NHR^1$ ou $NR^1R^2$ ($R^1$, $R^2$ = un radical aliphatique en $C_{1-4}$, cycloaliphatique en $C_{4-7}$, aliphatique/aromatique en $C_{6-8}$ ou aromatique/aliphatique en $C_{6-8}$), et $X^1$ et $X^2$ = H, un métal alcalin ou $NH_4$,

caractérisé en ce que

(a) on active sur la position 5' un diester préparé selon la revendication 2, étape (d2),

(b1) on fait réagir le diester activé sur un azide pour obtenir le diester de formule générale (I) (dans laquelle B est un résidu adénine, guanine, cytosine, thymine ou uracile, R est un radical azide et $R^1$ et $R^2$ sont chacun un radical alkyle en $C_{1-4}$); ou bien

(b2) on fait réagir le diester activé sur de l'ammoniac liquide ou sur une amine primaire ou secondaire pour obtenir le semi-ester de formule générale (I) (dans laquelle B est un résidu adénine, guanine, cytosine, thymine ou uracile, R est un radical $NH_2$, $NHR^1$ ou $NR^1R^2$, $R^1$ et $R^2$ sont chacun un radical aliphatique en $C_{1-4}$, cycloaliphatique en $C_{4-7}$, aliphatique/aromatique en $C_{6-8}$ ou aromatique/aliphatique en $C_{6-8}$, $X^1$ est un atome d'hydrogène, un métal alcalin ou $NH_4$, et $X^2$ est un radical alkyle en $C_{1-4}$); et éventuellement

(b3) on transestérifie à l'aide d'un halogénotriméthylsilane le diester ou le semi-ester obtenu, et on le saponifie en l'acide libre ou en l'un de ses sels de formule générale (I) (dans laquelle B est un résidu adénine, guanine, cytosine, thymine ou uracile, R est un radical $N_3$, NH, $NHR^1$ ou $NR^1R^2$, $R^1$ et $R^2$ sont chacun un radical aliphatique, en $C_{1-4}$, cycloaliphatique en $C_{4-7}$, aliphatique/aromatique en $C_{6-8}$ ou aromatique/aliphatique en $C_{6-8}$, et $X^1$ et $X^2$ sont chacun un atome d'hydrogène, un métal alcalin ou $NH_4$).

4. Procédé pour la préparation de composés de formule générale (I) selon la revendication 1, dans laquelle les symboles suivants ont les significations suivants:

B = adénine, guanine, cytosine, thymine ou uracile,
R = -O-PO($OY^1$)($OY^2$), -O-PS($OY^1$)($OY^2$), -O-PO($OY^1$)-O-PO($OY^2$)($OY^3$) ou -O-PO($OY^1$)-O-PO($OY^1$)-O-PO($OY^3$)($OY^4$)
($Y^1$, $Y^2$, $Y^3$ et $Y^4$ = H, un métal alcalin ou $NH_4$), et $X^1$ et $X^2$ = un groupe alkyle en $C_{1-4}$; ou bien

$X^1$ = H, un métal alcalin ou $NH_4$ et $X^2 = X^1$ ou un groupe alkyle en $C_{1-4}$,
caractérisé en ce que

(a) on phosphoryle un diester préparé selon la revendication 2, étape (d2), d'une manière connue en soi, avec $POCl_3$ ou $PSCl_3$ dans un phosphate de trialkyle pour obtenir le diester de formule générale (I), et on le saponifie en l'acide monophosphorique-5' ou thionophosphorique-5' ou leurs sels (où B est un résidu adénine, guanine, cytosine, thymine ou uracile, R est un résidu -O-$PO(OY^1)(OY^2)$ ou -O-$PS(OY^1)(OY^2)$, $Y^1$ et $Y^2$ sont chacun un atome d'hydrogène, un métal alcalin ou $NH_4$; et $X^1$ et $X^2$ sont chacun un radical alkyle en $C_{1-4}$); éventuellement

(b) on saponifie le produit obtenu en le semi-ester ou en l'acide méthylènephosphonique-3' libre de formule générale (I) (dans laquelle B est un résidu adénine, guanine, cytosine, thymine ou uracile, R est un radical -O-$PO(OY^1)(OY^2)$ ou -O-$PS(OY^1)(OY^2$, $X^1$, $Y^1$ et $Y^2$ sont chacun un atome d'hydrogène, un métal alcalin ou $NH_4$; et $X^2 = X^1$ ou un radical alkyle en $C_{1-4}$); et, éventuellement

(c) avant ou après l'étape (b), on procède d'une manière connue en soi à la phosphorylation, par voie chimique ou enzymatique, de l'acide phosphorique obtenu dans l'étape (a) ou de son sel pour obtenir les acides diphosphoriques ou triphosphoriques ou leurs sels de formule générale (I) (dans laquelle B est un résidu adénine, guanine, cytosine, thymine ou uracile, R est un radical O-$PO(OY^1)$-O-$PO(OY^2)(OY^3)$ ou -O-$PO(OY^1)$-O-$PO(OY^2)$-O-$PO(OY^3)(OY^4)$; $Y^1$, $Y^2$ $Y^3$ et $Y^4$ sont chacun un atome d'hydrogène, un métal alcalin ou $NH_4$, et $X^1$ est un atome d'hydrogène, un métal alcalin, $NH_4$ ou un radical alkyle en $C_{1-4}$, et $X^2$ est un radical alkyle en $C_{1-4}$).

5. Procédé pour la préparation de composés de formule générale (I) selon la revendication 1, dans laquelle les symboles suivants ont les significations suivantes:

B = adénine, guanine, cytosine, thymine ou uracile,

R et $X^2$ manquent avec formation d'un cycle par le groupement -$PO(OX^1)$-O-$CH_2$, $X^1$ = H, un métal alcalin, $NH_4$ ou un groupe alkyle en $C_{1-4}$,
caractérisé en ce que

(a) on fait réagir un diester préparé selon la revendication 2, étape (d2), sur des catalyseurs alcalins pour obtenir un composé de formule générale (I) (dans laquelle B est un résidu adénine, guanine, cytosine, thymine ou uracile, $X^1$ est un radical alkyle $C_{1-4}$, et R et $X^2$ manquent avec formation d'un cycle par le groupement -$PO(OX^1)$-O-$CH_2$-); ou bien

(b) on cyclise l'acide libre ou l'un de ses sels, préparé selon la revendication 2, étape (d1), avec des agents de cyclisation dans un solvant organique à haut température; et éventuellement

(c) on transestérifie le produit obtenu dans l'étape (a) à l'aide d'un halogénotriméthylsilane dans un hydrocarbure halogéné, et on le saponifie en l'acide libre ou ses sels de formule générale (I) (dans laquelle B est un résidu adénine, guanine, cytosine, thymine ou uracile, $X^1$ est un atome d'hydrogène, un métal alcalin ou $NH_4$, et R et $X^2$ manquet avec formation d'un cycle par le groupement -$PO(OX^1)$-O-$CH_2$-.

6. Procédé pour la préparation de composés de formule générale (I) selon la revendication 1, dans laquelle les symboles suivants ont les significations suivantes:

R = O-monométhoxytrityle-5' ou O-(diméthoxy-4,4'-trityle)-5',

B = $N^6$-benzoyladénine; $N^2$-isobutyrylguanine, $N^2$-isobutyryl, O-(p-nitrophényléthyl)-6-guanine; O-(p-nitrophényléthyl)-4-thymine; $N^4$-benzoylcytosine ou anisoyl-4-cytosine

$X^1$ = chloro-2-phényle, trichloro-2,2,2-éthyle ou cyanoéthyle,

$X^2$ = triéthylammonium,
caractérisé en ce que

(a) on fait réagir en présence de diméthylamino-4-pyridine, d'une manière connue en soi, un composé préparé selon la revendication 2, étape (d2), ayant la formule générale suivante 8

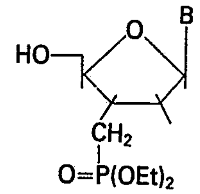
8

dans laquelle B a la signification indiquée, sur du chlorure de monométhoxy-4-triphénylméthyle (chlorure de méthoxytrityle),

(b) on fait réagir d'une manière connue en soi sur un halogénotriméthylsilane le composé obtenu de formule générale 9,

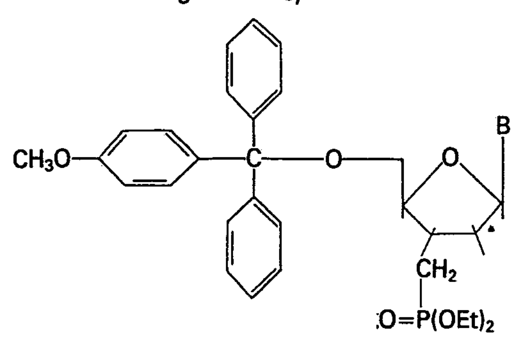
9

(c) on saponifie au méthanol le produit de réaction de formule générale 10

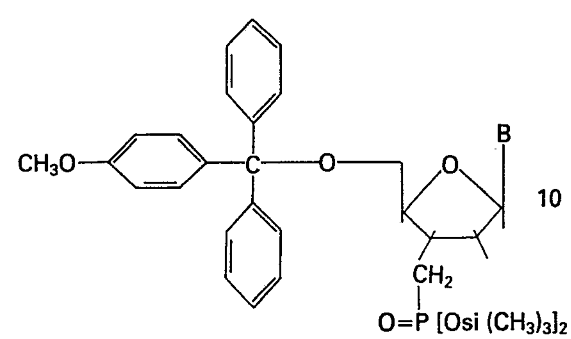
10

et on le chromatographie sur DEAE-Sephadex®,

(d) on estérifie d'une manière connue en soi, pour obtenir le composé de formule générale 7, le

produit de réaction de formule générale 11

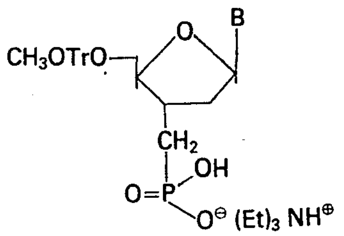

11

en utilisant du triisopropyl-2,4,6-benzènesulfochlorure dans de la pyridine (activation) avec du chloro-2-phénol.

**Claims**

1. 2′-Deoxy-3′-phosphonylmethyl-nucleosides of the following general formula

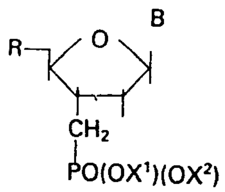

(I)

in which the following symbols have the following meanings:

B = adenine, $N^6$-benzoyladenine;
guanine, $N^2$-isobutyrylguanine, $N^2$-isobutyryl-6-O-(p-nitrophenylethyl)-guanine;
cytosine, $N^4$-benzoylcytosine, 4-anisoylcytosine;
thymine, 4-O-(p-nitrophenylethyl)-thymine; or uracil, and

(a) $X^1$, $X^2$ = H, alkali metal, $NH_4$ or $C_{1-4}$-alkyl, or
$X^1$ = chlorophenyl, 2,2,2-trichloroethyl or cyanoethyl, and
$X^2$ = triethylammonium,
R = OH, $N_3$, $NH_2$, $NHR^1$, $NR^1R^2$, -O-PO($OY^1$)($OY^2$), -O-PS($OY^1$)($OY^2$), -O-PO($OY^1$)-O-(PO($OY^2$)($OY^3$), -O-PO($OY^1$)-O-PO($OY^2$)-O-PO($OY^3$)($OY^4$),
$R^1$, $R^2$ being $C_{1-4}$-alkyl, $C_{4-7}$-cycloalkyl, $C_{6-8}$-alkylaryl or $C_{6-8}$-aralkyl and
$Y^1$, $Y^2$, $Y^3$, $Y^4$ being H, alkali metal or $NH_4$, or
R = 5′-O-monomethoxytriphenylmethyl (5′-O-monomethoxytrityl) or 5′-O-(4,4′-dimethoxytriphenylmethyl) (5′-O-(4,4′-dimethoxytrityl)) or
(b) $X_1$ = H, alkali metal, $NH_4$ or $C_{1-4}$-alkyl,
$X^2$ and R being absent with the formation of a ring of the grouping -PO($OX^1$)-O-$CH_2$-.

2. Process for the manufacture of the 2′-deoxy-3′-phosphonylmethyl-nucleosides of the following general formula (I) according to claim 1,

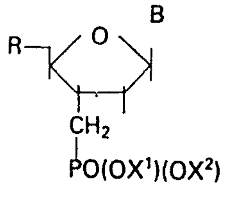

(I),

the following symbols having the following meanings:

— B = adenine, guanine, cytosine, thymine or uracil,
R = OH, $X^1$ and $X^2$ = H, alkali metal or $NH_4$; or
— B = adenine, guanine, cytosine, thymine or uracil,
R = OH, $X^1$ and $X^2$ = $C_{1-4}$-alkyl; or
— B = adenine, guanine, cytosine, thymine or uracil,
R = OH, $X^1$ = H, alkali metal or $NH_4$, and
$X^2$ = $C_{1-4}$-alkyl,

characterised in that

(a) from a compound of the general formula 1

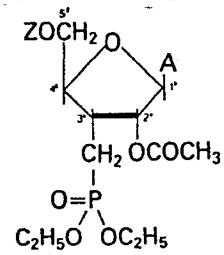

in which A is an N-protected adenine, guanine, cytosine, thymine or uracil radical and Z is benzoyl or monomethoxytrityl, the acetyl group is removed selectively in a manner known per se using an inorganic N-base or a salt thereof,

(b) the reaction product of the general formula 2

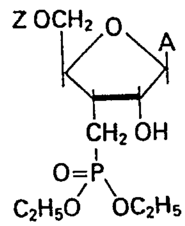

in which A is an optionally N-protected adenine, guanine, cytosine, thymine or uracil radical, is esterified in a manner known per se with a compound of the formula $R^3$-CS-$R^4$ ($R^3$ = Cl, $R^4$ = phenyl, phenoxy; $R^3$ = $R^4$ = imidazol-1-yl) in the presence of a 4-dialkylaminopyridine in an organic solvent,

(c) the resulting reaction product of the general formula 3

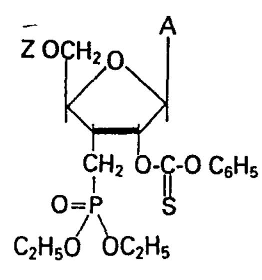

is reduced with tributyltin hydride in an inert aromatic solvent,

(d1) the reaction product of the general formula 4

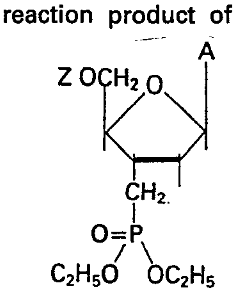

is transesterified with halotrimethylsilane in a halogenated hydrocarbon and hydrolysed to form the free acid or its ammonium or alkali metal salt of the general formula (I) ($X^1$ and $X^2$ representing a hydrogen atom, an alkali metal or $NH_4$ and R representing an OH group), or

(d2) the mentioned protecting group Z is removed from the reaction product of the general formula 4 with esterase (E.E. 3.1.1.1.) from pig's liver to form a diester of the general formula (I) ($X^1$ and $X^2$ representing a $C_{1-4}$-alkyl group and R representing an OH group), and, optionally,

(d3) the diester is hydrolysed to form a monoester of the general formula (I) ($X^1$ representing a hydrogen atom, an alkali metal or $NH_4$, $X^2$ representing a $C_{1-4}$-alkyl group and R representing an OH group).

3. Process for the manufacture of compounds of the general formula (I) according to claim 1, in which the following symbols have the following meanings:

— B = adenine, guanine, cytosine, thymine or uracil,

R = $N_3$ and $X^1$ and $X^2$ = $C_{1-4}$-alkyl; or

— B = adenine, guanine, cytosine, thymine or uracil,

R = $NH_2$, $NHR^1$ or $NR^1R^2$ ($R^1$, $R^2$ = $C_{1-4}$-aliphatic, $C_{4-7}$-cycloaliphatic, $C_{6-8}$-aliphatic/aromatic or $C_{6-8}$-aromatic/aliphatic radical),

$X^1$ = H, alkali metal or $NH_4$ and $X^2$ = $C_{1-4}$-alkyl; or

— B = adenine, guanine, cytosine, thymine or uracil,

R = $N_3$, $NH_2NHR^1$ or $NR^1R^2$ ($R^1$, $R^2$ = $C_{1-4}$-aliphatic, $C_{4-7}$-cycloaliphatic, $C_{6-8}$-aliphatic/aromatic or $C_{6-8}$-aromatic/aliphatic radical), and $X^1$ and $X^2$ = H, alkali metal or $NH_4$,

characterised in that

(a) a diester manufactured according to claim 2, stage (d2), is activated at the 5'-position,

(b1) the activated diester is reacted with an azide to form a diester of the general formula (I) (B representing an adenine, guanine, cytosine, thymine or uracil radical, R representing an azide radical and $R^1$ and $R^2$ representing a $C_{1-4}$-alkyl radical); or

(b2) the activated diester is reacted with liquid ammonia or with a primary or secondary amine to form a semiester of the general formula (I) (B representing an adenine, guanine, cytosine, thymine or uracil radical, R representing an $NH_2$, $NHR^1$ or $NR^1R^2$ radical, $R^1$ and $R^2$ representing a $C_{1-4}$-aliphatic, $C_{4-7}$-cycloaliphatic, $C_{6-8}$-aliphatic/aromatic or $C_{6-8}$-aromatic/aliphatic radical, $X^1$ representing a hydrogen atom, an alkali metal or $NH_4$ and $X^2$ representing a $C_{1-4}$-alkyl radical); and, optionally,

(b3) the resulting diester or semiester is transesterified with halotrimethylsilane and hydrolysed to form the free acid or a salt thereof of the general formula (I) (B representing an adenine, guanine, cytosine, thymine or uracil radical, R representing an $N_3$, $NH_2$, $NHR^1$ or $NR^1R^2$ radical, $R^1$ and $R^2$ representing a $C_{1-4}$-aliphatic, $C_{4-7}$-cycloaliphatic, $C_{6-8}$-aliphatic/aromatic or $C_{6-8}$-aromatic/aliphatic radical and $X^1$ and $X^2$ representing a hydrogen atom, an alkali metal or $NH_4$).

4. Process for the manufacture of compounds of the general formula (I) according to claim 1, in which the following symbols have the following meanings:

— B = adenine, guanine, cytosine, thymine or uracil,

R = -O-PO($OY^1$)($OY^2$), -O-PS($OY^1$)($OY^2$), -O-PO($OY^1$)-O-PO($OY^2$)($Y^3$) or -O-PO($OY^1$)-O-PO($OY^2$)-O-PO($OY^3$)($OY^4$)

($Y^1$, $Y^2$, $Y^3$ and $Y^4$ = H, alkali metal or $NH_4$ and $X^1$ and $X^2$ = $C_{1-4}$-alkyl; or

$X^1$ = H, alkali metal or $NH_4$ and $X^2$ = $X^1$ or $C_{1-4}$-alkyl,

characterised in that

(a) a diester manufactured according to claim 2, stage (d2), is phosphorylated in a manner known per se with $POCl_3$ and $PSCl_3$ in a phosphoric acid trialkyl ester to form a diester of the general formula (I) and is hydrolysed to form free 5'-monophosphoric or 5'-thionophosphoric acid or a salt thereof (B representing an adenine, guanine, cytosine, thymine or uracil radical, R representing a -O-PO($OY^1$)($OY^2$) or -O-PS($OY^1$)($OY^2$) radical, $Y^1$ and $Y^2$ representing a hydrogen atom, an alkali metal or $NH_4$, and $X^1$ and $X^2$ representing a $C_{1-4}$-alkyl radical); and, optionally,

(b) the resulting product is hydrolysed to form a semiester or to form free 3'-methylenephosphonic acid of the general formula (I) (B representing an adenine, guanine, cytosine, thymine or uracil radical, R representing a -O-PO($OY^1$)($OY^2$), or-O-PS($OY^1$)($OY^2$) radical, $X^1$, $Y^1$ and $Y^2$ representing a hydrogen atom, an alkali metal or $NH_4$ and $X^2$ = $X^2$ or a $C_{1-4}$-alkyl radical); and, optionally,

(c) before or after step (b), the phosphoric acid obtained in stage (a) or a salt thereof is phosphorylated chemically or enzymatically in a manner known per se to form di- or tri-phosphoric acids or salts thereof of the general formula (I) (B representing an adenine, guanine, cytosine, thymine or uracil radical, R representing a -O-PO($OY^1$)-O-PO($OY^2$)($OY^3$) or -O-PO($OY^1$)-O-PO($OY^2$)-O-PO($OY^3$)($OY^4$) radical, $Y^1$, $Y^2$, $Y^3$ and $Y^4$ representing a hydrogen atom, an alkali metal or $NH_4$, and $X^1$ representing a hydrogen atom, an alkali metal, $NH_4$ or a $C_{1-4}$-alkyl radical, and $X^2$ representing a $C_{1-4}$-alkyl radical).

5. Process for the manufacture of compounds of the general formula (I) according to claim 1, in which the following symbols have the following meanings:

— B = adenine, guanine, cytosine, thymine or uracil,

R and $X^2$ are absent with the formation of a ring of the grouping -PO($OX^1$)-O-$CH_2$-, $X^1$ = H, alkali metal, $NH_4$ or $C_{1-4}$-alkyl,

characterised in that

(a) a diester manufactured according to claim 2, stage (d2), is reacted with alkaline catalysts to form a compound of the general formula (I) (B representing an adenine, guanine, cytosine, thymine or uracil radical and $X^1$ representing a $C_{1-4}$-alkyl radical, and R and $X^2$ being absent with the

formation of a ring of the grouping -PO($OX^1$)-O-$CH_2$-); or

(b) the free acid manufactured according to claim 2, stage (d1), or a salt thereof, is cyclised with cyclisation agents in an organic solvent at elevated temperature; and optionally,

(c) the resulting product of stage (a) is transesterified with a halotrimethylsilane in a halogenated hydrocarbon and hydrolysed to form the free acid or a salt thereof of the general formula (I) (B representing an adenine, guanine, cytosine, thymine or uracil radical and $X^1$ representing a hydrogen atom, an alkali metal or $NH_4$, and R and $X^2$ being absent with the formation of a ring of the grouping -PO($OX^1$)-O-$CH_2$).

6. Process for the manufacture of compounds of the general formula (I) according to claim 1, in which the following symbols have the following meanings:

R = 5'-O-monomethoxytrityl or 5'-O-(4,4'-dimethoxytrityl),

B = $N^6$-benzoyladenine; $N^2$-isobutyrylguanine, $N^2$-isobutyryl-6-O-(p-nitrophenylethyl)-guanine; 4-O-(p-nitrophenylethyl)-thymine; $N^4$-benzoylcytosine or 4-anisoylcytosine,

$X^1$ = 2-chlorophenyl, 2,2,2-trichloroethyl or cyanoethyl,

$X^2$ = triethylammonium

characterised in that

(a) a compound of the following general formula 8 manufactured according to claim 2, stage (d2),

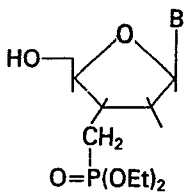

8

in which B has the meaning given, is reacted in a manner known per se with 4-monomethoxytriphenylmethyl-chloride (methoxytritylchloride) in the presence of 4-dimethylaminopyridine,

(b) the resulting compound of the general formula 9

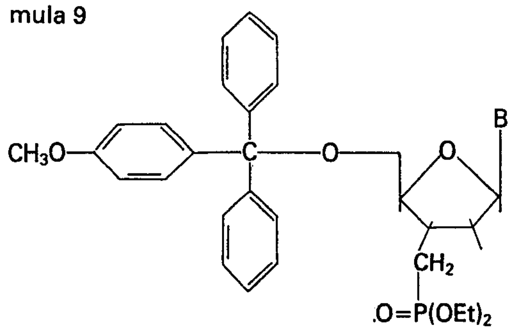

9

is reacted in a manner known per se with halotrimethylsilane,

(c) the reaction product of the general formula 10

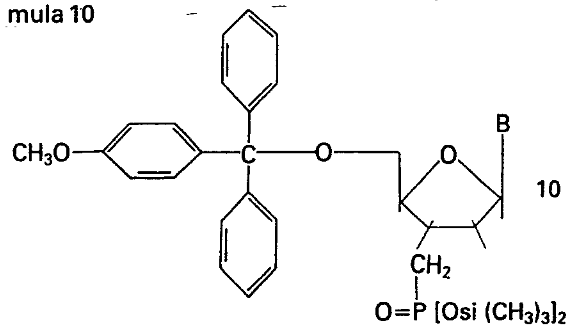

10

is hydrolysed with methanol and chromatographed on DEAE-Sephadex®,

(d) the reaction product of the general formula 11

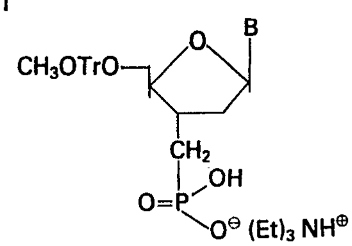

11

is esterified in a manner known per se using 2,4,6-triisopropylbenzenesulphochloride in pyridine (activation) with 2-chlorophenol to form a compound of the general formula 7.